# EUROPÄISCHE PATENTSCHRIFT

(11) **EP 2 956 021 B1**
(45) Veröffentlichungstag und Bekanntmachung des Hinweises auf die Patenterteilung: **18.10.2017**
(21) Anmeldenummer: 14710495.4
(22) Anmeldetag: 14.02.2014
(51) Int. Cl.: A24F 47/00, A61M 15/06, A61M 15/00

(54) **WIRKSTOFFDEPOT FÜR EINE INHALATIONSEINRICHTUNG**
ACTIVE INGREDIENT DEPOT FOR AN INHALATION DEVICE
DÉPÔT DE MATIÈRE ACTIVE POUR UNE INSTALLATION D'INHALATION

(30) Priorität: 14.02.2013 DE 102013002581
(43) Veröffentlichungstag der Anmeldung: 23.12.2015
(73) Patentinhaber: Similtrade GmbH, 6343 Rotkreuz (CH)
(72) Erfinder: SCHEIBER, Patrik, CH-8155 Niederhasli (CH); SPÖRRI, Anton, CH-6353 Weggis (CH); SPÖRRI, Markus, CH-6314 Unterägeri (CH)
(74) Vertreter: Weigel, Matthias
(86) Internationale Anmeldenummer: PCT/EP2014/000417
(87) Internationale Veröffentlichungsnummer: WO 2014/124756

(56) Entgegenhaltungen:
- DE-A1-102011 011 676
- US-A- 6 098 632
- ANONYMOUS: "Topas Cyclic Olefin Copolymer (COC)", INTERNET CITATION, März 2006 (2006-03), Seiten 1-20, XP002700428, Gefunden im Internet: URL:http://www.topas.com/sites/default/fil es/files/topas_product-brochure_english.pd f [gefunden am 2013-07-09]

## Beschreibung

Die Erfindung betrifft ein Wirkstoffdepot für eine Inhalationseinrichtung, mit einem vor der Benutzung nach außen hin luftdicht versiegelten Depotkörper, der zumindest einen aus einem Kunststoffmaterial gefertigten Abschnitt aufweist, mit einem zur Benutzung zu entsiegelnden Strömungskanal, und mit mindestens einem im Strömungskanal vorgesehenen Wirkstoffträger, in dem mindestens eine bei Erwärmung zumindest teilweise in die Gasphase übergehende flüchtige Substanz abgespeichert ist, wobei der Wirkstoffträger zur Freisetzung der Substanz zu erwärmen ist.

Seit geraumer Zeit kommen Inhalationseinrichtungen zum Einsatz, mit deren Hilfe Benutzer flüchtige Substanzen inhalieren können. Die Inhalationseinrichtung weist hierzu ein Wirkstoffdepot auf, aus dem die Substanz freigesetzt und an einen Luftstrom abgegeben wird, der vom Benutzer durch die Inhalationseinrichtung gesogen wird.

Derartige Inhalationseinrichtungen werden unter anderem für das inhalieren von pharmazeutisch wirksamen Substanzen verwendet, beispielsweise zur Behandlung von Hals- und Rachenschmerzen oder auch zur Behandlung von Lungenerkrankungen. Vorteil hierbei ist, dass mit vergleichsweise geringen technischen Mitteln eine definierte und sehr gezielte Abgabe der als Wirkstoffe dienenden flüchtigen Substanzen möglich ist. Um eventuell unangenehme Begleiterscheinungen zu eliminieren oder zu lindern, ist es auch möglich, neben den pharmazeutisch wirksamen Wirkstoffen zusätzliche Substanzen, wie schmerzlindernde Wirkstoffe, Aromastoffe und ähnliches, im Wirkstoffträger zu speichern, die gemeinsam mit der pharmazeutisch wirksamen Substanz freigesetzt werden.

Anstelle pharmazeutisch wirksamer Substanzen können als Wirkstoffe auch Nikotin oder nikotinhaltigen Verbindungen eingesetzt werden, die vom Benutzer konsumiert werden. Der maßgebliche Vorteil hierin liegt in der Tatsache, dass der Konsum erfolgen kann, ohne dass hierbei Tabak unter Rauchentwicklung verbrannt werden muss. Diese sogenannten rauchfreien Zigaretten, Zigarren oder Pfeifen sind als Inhalationseinrichtungen ausgebildet, mit deren Hilfe Wirkstoffe, wie Nikotin oder nikotinhaltigen Verbindungen, und auch Aromastoffe gezielt und definiert freigesetzt werden, die anschließend vom Benutzer inhaliert werden. Unter dem Begriff "nikotinhaltige Verbindung" werden in diesem Zusammenhang Nikotinsalze, Nikotinderivate sowie nikotinhaltige Tabakinhaltsstoffe verstanden.

Je nach Typ der Inhalationseinrichtung ist das Wirkstoffdepot entweder wesentlicher Bestandteil der Inhalationseinrichtung und wird gemeinsam mit dieser entsorgt, wenn keine Wirkstoffe mehr im Wirkstoffdepot gespeichert sind, oder das Wirkstoffdepot ist als Einmaldepot ausgelegt, das in eine wiederverwendbare Inhalationseinrichtung eingesetzt und nach Benutzung aus der Inhalationseinrichtung wieder entfernt und entsorgt wird.

Die Wirkstoffdepots werden üblicherweise in sehr großen Stückzahlen zentral gefertigt und anschließend ausgeliefert. Demzufolge müssen die Wirkstoffdepots so ausgebildet sein, dass sie sicher transportiert und gelagert werden können, ohne dass die im Wirkstoffdepot gespeicherten Wirkstoffe während des Transportes oder der Lagerung freigesetzt werden.

Um dies zu gewährleisten ist es deshalb üblich, für die Depotkörper der Wirkstoffdepots Materialien zu verwenden, die ausreichend gasdicht sind und ein Diffundieren der Wirkstoffe durch das Material aus dem Wirkstoffdepot verhindern. Ferner ist es üblich, die Wirkstoffdepots in Blisterverpackungen zusätzlich zu verpacken, um die Wirkstofffreisetzung zusätzlich zu minimieren.

Aus dem US-Patent US 6 098 632 ist ein Wirkstoffdepot mit einem nach außen hin luftdicht versiegelten Depotkörper bekannt. Der Depotkörper weist zumindest einen aus einem Kunststoffmaterial gefertigten Abschnitt auf, in dem ein zur Benutzung zu entsiegelnder Strömungskanal vorgesehen ist. Im Strömungskanal ist ein Wirkstoffträger aufgenommen, der bei Erwärmung eine zumindest teilweise in die Gasphase übergehende flüchtige Substanz, nämlich Nikotin, freisetzt, welche im Wirkstoffträger gespeichert ist. Als Kunststoffmaterial für den Depotkörper wird ein Acrylnitril Methyl Acrylat Copolymer verwendet, welches unter dem Handelsnamen Barex® vertrieben wird.

Der vorliegenden Erfindung liegt nun die Aufgabe zugrunde, ein Wirkstoffdepot anzubieten, das einerseits in sehr großen Stückzahlen hergestellt werden kann und gleichzeitig nach außen hin so sicher versiegelt ist, dass eine unbeabsichtigte Wirkstofffreisetzung wirksam verhindert ist.

Erfindungsgemäß wird diese Aufgabe durch ein Wirkstoffdepot mit den Merkmalen nach Anspruch 1 und insbesondere dadurch gelöst, dass das Kunststoffmaterial, aus dem der Abschnitt des Depotkörpers gefertigt ist, ein Polymer aus der Gruppe der Cyclo-Olefin-Copolymere ist.

Die Erfindung beruht darauf, dass aus einer nahezu unendlichen Anzahl an möglichen Werkstoffen ein Kunststoffmaterial ausgewählt wurde, das unterschiedlichste, zum Teil sich sogar entgegenstehende Eigenschaften aufweist.

So soll der Kunststoff einerseits sehr gut und mit geringem technischen Aufwand zu verarbeiten sein, andererseits gegenüber den im Wirkstoffträger gespeicherten Substanzen nicht nur beständig sein, sondern auch ein unbeabsichtigtes Herausdiffundieren der im Wirkstoffdepot gespeicherten Substanzen aus dem Wirkstoffdepot wirksam verhindern.

Kunststoffmaterialen, die gut zu verarbeiten sind, so Thermoplaste, die sich für das Spritzgießen eignen, sind langläufig bekannt. Die bekannten spritzgießfähigen Kunststoffe zeichnen sich jedoch üblicherweise dadurch aus, dass sie nur eingeschränkt gegenüber bestimmten Substanzen beständig sind oder aber, auch wenn sie beständig sind, eine zu hohe Wasserdampfdurchlässigkeit aufweisen, so dass sie für den Einsatz in den erfindungsgemäßen Wirkstoffdepots normalerweise ausscheiden.

Kunststoffe, die dagegen eine ausreichende Beständigkeit und eine möglichst geringe Substanzdurchlässigkeit aufweisen, sind gleichfalls bekannt, sind aber üblicherweise nur schwer zu verarbeiten und insbesondere für das Spritzgießen ungeeignet.

Ein drittes zu berücksichtigendes Kriterium bei der Auswahl eines geeigneten Kunststoffmaterials ist, dass das Werkstoffdepot nach dem Befüllen gasdicht versiegelt werden muss. Das Versiegeln muss dabei so erfolgen, dass eine absolut gasdichte Verbindung zwischen dem Kunststoffmaterial des Depotkörpers und dem Versiegelungsmaterial sichergestellt ist. Unabhängig hiervon dürfen die beim Versiegeln auftretenden Auswirkungen und Belastungen auf das Wirkstoffdepot, beispielsweise Temperatureinwirkung beim Heißsiegeln, nur so moderat sein, dass die im Werkstoffdepot vor dem Versiegeln bereits enthaltenen, leicht flüchtigen Substanzen durch den Siegelvorgang nicht unbeabsichtigt freigesetzt werden und während des Versiegelns zumindest teilweise entweichen.

Zur Lösung dieser verschiedenen Vorgaben, wurde nun erfindungsgemäß ein Polymer aus der Gruppe der Cyclo-Olefin-Copolymere als Kunststoffmaterial für das Werkstoffdepot ausgewählt.

Dieses Polymer ist gut zu verarbeiten, beispielsweise durch Spritzgießen, so dass auf sehr einfache Weise hohe Stückzahlen bei moderaten Kosten und komplexen Geometrien gefertigt werden können. Gleichzeitig ist das Polymer aber auch gegenüber einer Vielzahl Substanzen beständig und weist eine äußert geringe Wasserdampfdurchlässigkeit auf.

Ein weiterer überraschender Vorteil besteht darin, dass sich das Polymer entgegen der weitläufigen Meinung der Fachwelt mit geringem Aufwand und geringsten Auswirkungen und Belastungen auf das Polymer sehr gut mit geeigneten Folienmaterialen gasdicht verbinden lässt.

Erst durch die Kombination dieser drei wesentlichen Eigenschaften ist es überhaupt möglich, Wirkstoffdepots in ausreichender Stückzahl herzustellen, die gegenüber den in den Wirkstoffdepots abgespeicherten Substanzen nicht nur beständig sind und eine zu vernachlässigende Wasserdampfdurchlässigkeit aufweisen, sondern darüber hinaus auch noch ohne großen Aufwand nach dem Befüllen mit den leichtflüchtigen Substanzen zu versiegeln sind.

Bei dem erfindungsgemäßen Wirkstoffdepot wird nun vorgeschlagen, zumindest den Abschnitt des Depotkörpers, der die zu versiegelnde Öffnung aufweist, die mit dem Strömungskanal in Verbindung steht, aus einem Polymer aus der Gruppe der Cyclo-Olefin-Copolymere herzustellen. Das Polymer ist nicht nur beständig gegenüber einer Vielzahl flüchtiger Substanzen, sondern weist eine vergleichsweise geringe Wasserdampfdurchlässigkeit und lässt sich überraschend gut mit Folien versiegeln. Vor Benutzung muss die Folie dann nur noch entfernt oder durchstoßen werden, wobei unter dem Begriff "Durchstoßen" natürlich auch ein Durchstechen, ein Durchlöchern oder auch ein Aufstechen mit oder ohne Verwendung eines Werkzeuges verstanden wird.

Des weiteren eignet sich das Polymer aus der Gruppe der Cyclo-Olefin-Copolymere zum Spritzgießen. Vorzugsweise ist deshalb der aus dem Cyclo-Olefin-Copolymer gefertigte Abschnitt des Depotkörpers ein Spritzgußteil.

Weitere Vorteile der Erfindung ergeben sich aus der nachfolgenden Beschreibung, den Unteransprüchen und den Zeichnungen.

Um zu verhindern, dass die im Wirkstoffdepot gespeicherte flüchtige Substanz möglichst nicht aus dem Depotkörper diffundiert, sollte die Wasserdampfdurchlässigkeit des Polymers möglichst gering sein. Aus diesem Grund wird vorgeschlagen, für den Depotkörper ein Polymer zu verwenden, dessen Wasserdampfdurchlässigkeit gemäß DIN 53122-2 (bei einer Umgebungstemperatur von 23°C und einer relativen Feuchte von 85%) in einem Bereich von 0,02 bis 0,05 g mm/m² d, vorzugsweise in einem Bereich von 0,03 bis 0,04 g mm/m² d, besonders bevorzugt bei 0,035 g mm/m² d liegt.

Damit eine Vielzahl flüchtiger Substanzen mit völlig unterschiedlichstem chemischen Verhalten im Wirkstoffdepot gespeichert werden können, sollte ein Polymer aus der Gruppe der Cyclo-Olefin-Copolymere für den Depotkörper verwendet werden, das gegen Hydrolyse, Säuren, Laugen und polare organische Lösungsmittel beständig ist.

Um eine problemlose Weiterverarbeitung des aus dem Polymer gefertigten Abschnittes des Depotkörpers zu ermöglichen, wird ferner vorgeschlagen, ein Polymer aus der Gruppe der Cyclo-Olefin-Copolymere zu verwenden, das nicht zu Sprödbruch neigt, sondern eine ausreichend hohe Zähigkeit aufweist. Aus diesem Grund wird vorgeschlagen, ein Polymer zu verwenden, dessen Schlagzähigkeit gemäß ISO 179/1 eU (Prüfkörper 80mm x 10mm x 4mm, Schlagrichtung - schmalseitig, Prüfkörper ungekerbt) in einem Bereich von 10 bis 25 kJ/m², vorzugsweise in einem Bereich von 15 bis 20 kJ/m², besonders bevorzugt bei 15 kJ/m² liegt.

Um auch die Auswirkungen kleinerer Vertiefungen, Kerben oder oberflächlichen Beschädigungen zu minimieren, sollte ein Polymer verwendet werden, dessen Kerbschlagzähigkeit gemäß ISO 179/1 eA (Prüfkörper 80mm x 10mm x 4mm, Schlagrichtung - schmalseitig, Prüfkörper mit Kerbe mit 0,25mm Radius) in einem Bereich von 3,0 bis 1,0 kJ/m², vorzugsweise in einem Bereich von 1,6 bis 2,6 kJ/m², besonders bevorzugt bei 1,8 kJ/m² liegt.

Ein weiterer wesentlicher Gedanke der Erfindung beruht auf der Verwendung einer Folie, mit der die Öffnung an dem aus dem Polymer gefertigten Abschnitt des Depotkörpers versiegelt werden soll. Entgegen der Auffassung der Fachleute wurde als Folie eine Metallfolie verwendet, die durch Heißsiegeln gasdicht mit dem Öffnungsumfang des Depotkörpers verbwunden wird, wobei das Polymer, anders als angenommen, eine gasdichte und innige Verbindung mit der Heißsiegelbeschichtung der Metallfolie eingeht. Auf diese Weise ist die Öffnung am Depotkörper nach außen hin gut abgedichtet, wobei die beim Versiegeln entstehenden Erwärmungen zum Heißsiegeln so moderat sind, dass ein unbeabsichtigtes Freisetzen der flüchtigen Substanzen aus dem Wirkstoffträger nicht auftritt. Versuche haben gezeigt, dass die Siegelung Druckdifferenzen von bis zu 0,3 bar unbeschadet übersteht.

Besonders von Vorteil ist bei dieser Ausführungsform die Verwendung einer Metallfolie, vorzugsweise eine Aluminiumfolie, die eine Materialdicke in einem Bereich von 20 bis 40 µm, vorzugsweise eine Materialdicke in einem Bereich von 25 bis 35 µm, besonders bevorzugt eine Materialdicke von 30 µm aufweist, während die mit dem Depotkörper versiegelte Flachseite der Folie mit einer Heißsiegellackschicht versehen ist, mit der die Metallfolie mit dem Polymer des Abschnittes des Depotkörpers gasdicht verbunden ist.

Durch die vergleichsweise geringe Materialdicke kann sehr schnell und gezielt Hitze auf die mit dem Depotkörper zu verschmelzende Heißsiegelbeschichtung übertragen werden, so dass das Versiegeln der Öffnung sehr schnell und ohne große Wärmeeinwirkung auf den Depotkörper erfolgt. Gleichzeitig wirkt das Polymer, aus dem der Depotkörper zumindest abschnittsweise gefertigt ist, wärmeisolierend, so dass die beim Heißsiegeln eingebrachte Wärmemenge nicht auf den Wirkstoffträger übertragen wird und somit ein unbeabsichtigtes Freisetzen der flüchtigen Substanzen aus dem Wirkstoffträger vermieden wird.

Bei einer besonders bevorzugten Ausführungsform des erfindungsgemäßen Wirkstoffdepots weist der Depotkörper zwei aus dem Polymer gefertigte Abschnitte auf, zwischen denen eine Metallhülse angeordnet ist, deren freie Enden mit den aus dem Polymer gefertigten Abschnitten gasdicht verbunden sind. In der Metallhülse ist der mindestens eine Wirkstoffträger gehalten, in dem die flüchtigen Substanzen gespeichert sind, Die Metallhülse dient als Wärmeübertragungselement und überträgt bei Benutzung die Körperwärme des Benutzers auf den Wirkstoffträger, so dass die im Wirkstoffträger gespeicherte Substanz an die durch den Benutzer durch den Strömungskanal angesaugten Luft freigesetzt wird.

Diese bevorzugte Ausführungsform hat den Vorteil, dass durch die mittige Anordnung der Metallhülse zwischen den aus Polymer gefertigten Abschnitten des Depotkörpers die Wärme sehr gezielt auf den Wirkstoffträger überträgt wird, während die aus Polymer gefertigten Abschnitte wärmeisolierend wirken und so zumindest ein wesentlicher Teil der über die Metallhülse eingebrachten Wärmemenge an den Wirkstoffträger übertragen wird.

Dieses insbesondere für passive Inhalationseinrichtungen vorgesehene Wirkstoffdepot hat den Vorteil, dass die für das Freisetzen der flüchtigen Substanz notwendige Wärme lediglich über die Körperwärme des Benutzers in das Wirkstoffdepot eingebracht wird. Bei Verwendung hält der Benutzer das Wirkstoffdepot im Bereich der nach außen hin zugänglichen Metallhülse und erwärmt mit seiner über die Finger übertragenen Körpertemperatur die im Wirkstoffträger gespeicherten Substanzen. Die Wärmeleitfähigkeit sowie die Wärmekapazität der Metallhülse sind dabei so ausgelegt, dass die Körpertemperatur des Benutzers ausreicht, den Wirkstoffträger soweit zu erwärmen, dass die im Wirkstoffträger gespeicherten Substanzen freigesetzt werden. Da der Wirkstoffträger im Strömungskanal des Wirkstoffdepots angeordnet ist, durch den der Benutzer Luft ansaugt, werden die aus dem Wirkstoffträger freigesetzten Substanzen an den Luftstrom abgegeben.

Bei einer besonders bevorzugten Variante dieser Ausführungsform sind beide aus dem Polymer gefertigten Abschnitte des Depotkörpers jeweils mit einer mit dem Strömungskanal in Verbindung stehenden Öffnung versehen, die jeweils durch eine mit dem jeweiligen Abschnitt des Depotkörpers durch Heißsiegelung gasdicht verbundene Siegelfolie, welche unmittelbar vor Benutzung zu entfernen oder zu durchlöchern ist, versiegelt sind.

Nachfolgend wird die Erfindung anhand eines Ausführungsbeispiels unter Bezugnahme auf die Zeichnung näher erläutert. Hierin zeigt
- Fig. 1: eine Schnittansicht durch ein Ausführungsbeispiel einer Inhalationseinrichtung mit einem erfindungsgemäßen Wirkstoffdepot, und
- Fig. 2: einen vergrößerten Ausschnitt aus Fig. 1, in dem der Übergang zwischen einer Metallhülse und einer Hülse aus einem Polymer gezeigt ist.

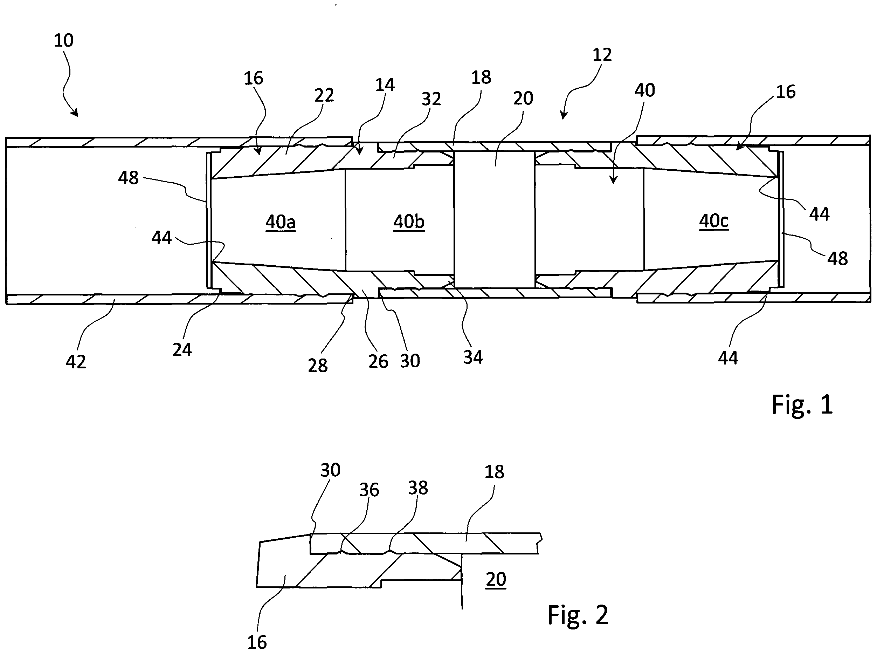

Fig. 1 zeigt eine Schnittansicht eines Ausführungsbeispiels einer Inhalationseinrichtung 10, die ein erfindungsgemäßes Wirkstoffdepot 12 aufweist.

Das Wirkstoffdepot 12 weist einen Depotkörper 14 auf. Der Depotkörper 14 hat zwei identisch ausgebildete Hülsen 16, zwischen denen eine Metallhülse 18 angeordnet ist. Die Metallhülse 18 ist in Form eines Hohlzylinders ausgeführt und aus einem Werkstoff mit sehr hoher Wärmeleitfähigkeit gefertigt, beispielsweise aus Aluminium oder aus einer Aluminiumlegierung.

In der Metallhülse 18 ist ein Wirkstoffträger 20 aufgenommen, in dem mindestens eine bei Raumtemperatur flüchtige Substanz, beispielsweise ein freizusetzender Wirkstoff, ein Aromastoff oder ein Geschmacksstoff, gespeichert ist. Der Wirkstoffträger 20 ist in Form eines Zylinders ausgebildet und steht mit der Innenoberfläche der Metallhülse 18 in Berührung. Im vorliegenden Fall dient der Wirkstoffträger 20 zum Abspeichern einer vorgegebenen Menge einer pharmazeutisch wirksamen Substanz.

Aus dem durchschnittlichen Zugvolumen von 35 ml und der Zugzeit eines durchschnittlichen Benutzers von 1 bis 2 Sekunden ergibt sich eine sehr kurze Kontaktzeit der den Wirkstoffträger 20 durchströmenden Luft mit der im Wirkstoffträger 20 gespeicherten pharmazeutisch wirksamen Substanz. Dabei korreliert der Übergang der freizusetzenden Substanz in die Gasphase in etwa mit der Kontaktfläche zwischen dem Wirkstoffträger 20 und der Luft.

Der gezeigte Wirkstoffträger 20 ist so in der Metallhülse 18 aufgenommen, dass die Metallhülse 18 beim Halten der Inhalationseinrichtung 10 mit der Hand des Benutzers unmittelbar in Berührung ist. Die Handwärme wird dann an die Metallhülse 18 übertragen, welcher ihrerseits die Wärme nach innen an den Wirkstoffträger 20 weiterleitet. Durch das Erwärmen des Wirkstoffträgers 20 wird die Freisetzungsrate der flüchtigen Substanz sowie gegebenenfalls im Wirkstoffträger 20 gespeicherter weiterer Substanzen, beispielsweise Aromastoffe, so erhöht, dass bei jedem Zug eine ausreichende Menge der flüchtigen Substanz und der Aromastoffe vom Benutzer aufgenommen werden kann. Das Volumen der Metallhülse 18 wird über deren Wandstärke so angepasst, dass die Aufwärmzeit nach der Positionierung zwischen den Fingern möglichst schnell verläuft.

In jedes offene Ende der Metallhülse 18 ist jeweils eine der beiden aus Kunststoff gefertigten Hülse 16 eingesetzt. Die beiden Hülsen 16 sind identisch ausgebildet, so dass nachfolgend nur die in Fig. 1 links gezeigte Hülse 16 näher beschrieben wird.

Die rotationssymmetrische Hülse 16 hat an ihrem in Fig. 1 links gezeigten Ende einen Abschnitt 22 mittleren Außendurchmessers. Das in Fig. 1 links dargestellte Ende des Abschnittes 22 ist mit einer Fase 24 versehen.

An den Abschnitt 22, der sich etwa über zwei Drittel der axialen Länge der Hülse 16 erstreckt, schließt sich ein umlaufender Bund 26 größeren Durchmessers an, der sich radial nach außen erstreckt. Der Bund 26 hat in axialer Richtung gesehen eine in Fig. 1 links dargestellte erste Anlagefläch 28 und eine in Fig. 1 rechts dargestellte zweite Anlagefläche 30.

Die zweite Anlagefläche 30 des Bundes 26 geht in einen Absatz 32 kleineren Durchmessers über. Das freie Ende des Absatzes 32 ist gleichfalls mit einer Fase 34 versehen. Die Hülse 16 ist mit dem Absatz 32 in die hohlzylindrische Metallhülse 18 soweit eingepresst, dass die Metallhülse 18 mit ihrer Stirnseite an der zweiten Anlagefläche 30 des Bundes 26 anliegt. Der Außendurchmesser des Absatzes 32 ist dabei so bemessen, dass der Absatz 32 mit der Innenumfangsfläche der Metallhülse 18 eine Presspassung ausbildet.

Fig. 2 zeigt einen vergrößerten Ausschnitt aus Fig. 1. Wie Fig. 2 zu entnehmen ist, ist der Absatz 32 der Hülse 16 zusätzlich mit zwei umlaufenden Dichtungsbunden 36 und 38 versehen, welche unter Vorspannung an der Innenumfangsfläche der Metallhülse 18 anliegen, um die Metallhülse 18 mit der Hülse 16 gasdicht zu verbinden. In entsprechender Weise ist auch die in Fig. 1 rechts gezeigte, zur links dargestellten Hülse 16 identisch ausgebildete Hülse 16 mit der Metallhülse 14 gasdicht verbunden.

Die axiale Länge des Absatzes 34 jeder Hülse 16 ist so gewählt und auf die axiale Länge des Depots 18 abgestimmt, dass das Depot 18 bei zusammengesetzter Inhalationseinrichtung 10 mittig in der Wärmeübertragungshülse 14 angeordnet zwischen den Absätzen 34 der beiden Hülsen 16 eingespannt, zumindest aber zwischen diesen angeordnet ist.

Durch die Inhalationseinrichtung 10 erstreckt sich ferner ein Strömungskanal 40. Der Strömungskanal 40 ist in mehrere Abschnitte 40a bis 40c unterteilt, nämlich einen in der ersten Hülse 16 ausgebildeten sich hohlkegelförmig erweiternden ersten Abschnitt 40a, einen in der Metallhülse 18 ausgebildeten hohlzylindrischen zweiten Abschnitt 40b, in dem der Wirkstoffträger 20 aufgenommen ist, sowie einen in der zweiten Hülse 16 ausgebildeten sich hohlkegelförmig verjüngenden dritten Abschnitt 40c.

Auf das freie Ende des Abschnittes 22 mittleren Durchmessers der in Fig. 1 links gezeigten Hülse 16 ist ein aus Papier gefertigtes Ansaugrohr 42 aufgesteckt. Auf das freie Ende des Abschnittes 22 mittleren Durchmessers der in Fig. 1 rechts gezeigten Hülse 16 ist ein aus Papier gefertigtes Mundstück 44 aufgesteckt. Sowohl das Ansaugrohr 42 als auch das Mundstück 44 sind beide gleichfalls als Hohlzylinder ausgebildet und stehen mit dem Strömungskanal 40 in Strömungsverbindung.

Die beiden identisch ausgebildeten Hülsen 16 sind als Spritzgußteile ausgeführt und bestehen aus einem Polymer aus der Gruppe der Cyclo-Olefin-Copolymere. Das für die beiden Hülsen 16 verwendete Polymer ist gegen Hydrolyse, Säuren, Laugen und polare organische Lösungsmittel beständig und weist eine Wasserdampfdurchlässigkeit in einem Bereich von 0,03 bis 0,04 g mm/m² d auf. Die Wasserdampfdurchlässigkeit wurde gemäß der DIN 53122-2 bei einer Umgebungstemperatur von 23°C und einer relativen Feuchte von 85% bestimmt.

Wie Fig. 1 weiter zu entnehmen ist, ist die Öffnung 46 am freien Ende jeder Hülse 16 durch eine Metallfolie 48 versiegelt. Die Metallfolie 48 besteht aus Aluminium und hat eine Materialdicke von 30 µm. An ihrer der Hülse 16 zugewandten Flachseite ist die Metallfolie 48 mit einem Heißsiegellack versehen, mit dem die Metallfolie mit der Hülse durch Heißversiegeln gasdicht verbunden worden ist.

Zur Benutzung der Inhalationseinrichtung 10 muss die Metallfolie 48 an beiden Hülsen 16 lediglich durchstoßen oder durchlöchert werden, so dass der Benutzer Luft durch den Strömungskanal 40 ansaugen kann.

### Bezugszeichenliste:

- 10: Inhalationseinrichtung
- 12: Wirkstoffdepot
- 14: Depotkörper
- 16: Hülsen
- 18: Metallhülse
- 20: Wirkstoffträger
- 22: Abschnitt mittleren Durchmessers
- 24: Fase
- 26: Bund
- 28: erste Anlagefläche
- 30: zweite Anlagefläche
- 32: Absatz kleineren Durchmessers
- 34: Fase
- 36: Dichtungsbund
- 38: Dichtungsbund
- 40: Strömungskanal
- 40a: erster Abschnitt des Strömungskanals
- 40b: zweiter Abschnitt des Strömungskanals
- 40c: dritter Abschnitt des Strömungskanals
- 42: Ansaugrohr
- 44: Mundstück
- 46: Öffnung
- 48: Metallfolie

## Patentansprüche

1. Wirkstoffdepot für eine Inhalationseinrichtung (10), mit einem vor der Benutzung nach außen hin luftdicht versiegelten Depotkörper (14), der zumindest einen aus einem Kunststoffmaterial gefertigten Abschnitt (16) aufweist, mit einem zur Benutzung zu entsiegelnden Strömungskanal (40), und mit mindestens einem im Strömungskanal (40) vorgesehenen Wirkstoffträger (20), in dem mindestens eine bei Erwärmung zumindest teilweise in die Gasphase übergehende flüchtige Substanz abgespeichert ist, wobei der Wirkstoffträger (20) zur Freisetzung der Substanz zu erwärmen ist, wobei das Kunststoffmaterial, aus dem der Abschnitt (16) des Depotkörpers (14) gefertigt ist, ein Polymer ist, und dass der aus dem Polymer gefertigte Abschnitt (16) des Depotkörpers (14) eine mit dem Strömungskanal (40) in Verbindung stehende Öffnung (46) aufweist, die durch eine aus einem zweiten Material bestehende Folie (48) versiegelt ist, welche unmittelbar vor Benutzung zu entfernen oder zu durchlöchern ist, **dadurch gekennzeichnet, dass** das Polymer aus der Gruppe der Cyclo-Olefin-Copolymere ist.

2. Wirkstoffdepot nach Anspruch 1, **dadurch gekennzeichnet, dass** das Polymer eine Wasserdampfdurchlässigkeit gemäß DIN 53122-2 (bei einer Umgebungstemperatur von 23°C und einer relativen Feuchte von 85%) in einem Bereich von 0,02 bis 0,05 g mm/m² d, vorzugsweise in einem Bereich von 0,03 bis 0,04 g mm/m² d, besonders bevorzugt bei 0,035 g mm/m² d liegt.

3. Wirkstoffdepot nach Anspruch 1, **dadurch gekennzeichnet, dass** das Kunststoffmaterial, aus dem der Abschnitt (16) des Depotkörpers (14) gefertigt ist, gegen Hydrolyse, Säuren, Laugen und polare organische Lösungsmittel beständig ist.

4. Wirkstoffdepot nach Anspruch 1, 2 oder 3, **dadurch gekennzeichnet, dass** die Schlagzähigkeit des Polymers, aus dem der Abschnitt (16) des Depotkörpers (14) gefertigt ist, gemäß ISO 179/1eU (Prüfkörper 80mm x 10mm x 4mm, Schlagrichtung - schmalseitig, Prüfkörper ungekerbt) in einem Bereich von 10 bis 25 kJ/m², vorzugsweise in einem Bereich von 15 bis 20 kJ/m², besonders bevorzugt bei 15 kJ/m² liegt.

5. Wirkstoffdepot nach Anspruch 1, 2 oder 3, **dadurch gekennzeichnet, dass** die Kerbschlagzähigkeit des Polymers, aus dem der Abschnitt (16) des Depotkörpers (14) gefertigt ist, gemäß ISO 179/1eA (Prüfkörper 80mm x 10mm x 4mm, Schlagrichtung - schmalseitig, Prüfkörper mit Kerbe mit 0,25mm Radius) in einem Bereich von 3,0 bis 1,0 kJ/m², vorzugsweise in einem Bereich von 1,6 bis 2,6 kJ/m², besonders bevorzugt bei 1,8 kJ/m² liegt.

6. Wirkstoffdepot nach einem der vorhergehenden Ansprüche, **dadurch gekennzeichnet, dass** die an dem aus dem Polymer gefertigten Abschnitt (16) des Depotkörpers (14) vorgesehene Öffnung (46) mit Hilfe einer durch Heißsiegelung gasdicht mit dem Polymer verbundene Metallfolie (48) versiegelt ist, welche unmittelbar vor Benutzung zu entfernen oder zu durchlöchern ist.

7. Wirkstoffdepot nach Anspruch 6, **dadurch gekennzeichnet, dass** die Metallfolie, vorzugsweise eine Aluminiumfolie (48), eine Materialdicke in einem Bereich von 20 bis 40 µm, vorzugsweise eine Materialdicke in einem Bereich von 25 bis 35 µm, besonders bevorzugt eine Materialdicke von 30 µm aufweist und auf einer ihrer Flachseiten mit einer Heißsiegellackschicht versehen ist, mit der die Metallfolie (48) mit dem Polymer des Abschnittes (16) des Depotkörpers (14) gasdicht verbunden ist.

8. Wirkstoffdepot nach einem der vorhergehenden Ansprüche, **dadurch gekennzeichnet, dass** der Depotkörper (14) zwei aus dem Kunststoffmaterial gefertigte Abschnitte (16) aufweist, zwischen denen eine Metallhülse (18) angeordnet ist, deren freie Enden mit den Abschnitten (16) gasdicht verbunden ist, dass in der Metallhülse (18) der mindestens eine Wirkstoffträger (12) gehalten ist, und dass die Metallhülse (18) als Wärmeübertragungselement dient und bei Benutzung die Körperwärme des Benutzers auf den Wirkstoffträger (20) derart überträgt, dass die im Wirkstoffträger (20) gespeicherte Substanz an die durch den Benutzer durch den Strömungskanal (40) angesaugten Luft freigesetzt wird.

9. Wirkstoffdepot nach Anspruch 8, **dadurch gekennzeichnet, dass** die beiden aus Kunststoffmaterial gefertigten Abschnitte (16) des Depotkörpers (14) jeweils eine mit dem Strömungskanal (40) in Verbindung stehende Öffnungen (46) aufweisen, die jeweils durch eine mit dem jeweiligen Abschnitt (16) des Depotkörpers (14) durch Heißsiegelung gasdicht verbundene Siegelfolie (48), welche unmittelbar vor Benutzung zu entfernen oder zu durchlöchern ist, versiegelt sind.

## Claims

1. An active substance depot for an inhalation device (10) having a depot body (14) externally sealed in an airtight manner prior to use, comprising at least one segment (16) made from a plastic material, having a flow channel (40) to be unsealed for use, and having at least one active substance carrier (20) provided in the flow channel (40) in which is stored at least one volatile substance at least partially transitioning into the gaseous phase when heated, the active substance carrier (20) being heated for releasing the substance, wherein the plastic material of which the segment (16) of the depot body (14) is made is a polymer, and that the segment (16) of the depot body (14) made from the polymer comprises an opening (46) connected to the flow channel (40), said opening being sealed by a foil (48) made of a second material, and said foil being removed or punctured immediately prior to use,
**characterized in that**
the polymer is from the group of cyclo olefin copolymers.

2. The active substance depot according to claim 1, **characterized in that** the polymer comprises a water vapor permeability according to DIN 53122-2 (at an ambient temperature of 23°C and relative humidity of 85%) in a range from 0.02 to 0.05 g mm/m² d, preferably in a range from 0.03 to 0.04 g mm/m² d, particularly preferably of 0.035 g mm/m² d.

3. The active substance depot according to claim 1, **characterized in that** the plastic material of which the segment (16) of the depot body (14) is made is resistant to hydrolysis, acids, bases, and polar organic solvents.

4. The active substance depot according to claim 1, 2, or 3, **characterized in that** the impact strength of the polymer of which the segment (16) of the depot body (14) is made is within a range from 10 to 25 kJ/m², preferably in a range from 15 to 20 kJ/m², particularly preferably of 15 kJ/m², according to ISO 179/1 eU (test body 80mm x 10mm x 4mm, narrow side impact direction, unnotched test body).

5. The active substance depot according to claim 1, 2, or 3, **characterized in that** the notch impact strength of the polymer of which the segment (16) of the depot body (14) is made is within a range from 3.0 to 1.0 kJ/m², preferably in a range from 1.6 to 2.6 kJ/m², particularly preferably of 1.8 kJ/m², according to ISO 179/1 eU (test body 80mm x 10mm x 4mm, narrow side impact direction, test body notched with 0.25mm radius).

6. The active substance depot according to any one of the preceding claims, **characterized in that** the opening provided on the segment (16) of the depot body (14) made of the polymer is sealed gas-tight by means of a metal foil (48) bonded to the polymer by hot sealing, said foil being removed or punctured immediately prior to use.

7. The active substance depot according to claim 6, **characterized in that** the metal foil, preferably an aluminum foil (48), comprises a material thickness in a range from 20 to 40 µm, preferably a material thickness in a range from 25 to 35 µm, particularly preferably a material thickness of 30 µm, and has a heat sealing lacquer coating on one of the flat sides thereof, by means of which the metal foil (48) is bonded in a gas-tight manner to the polymer of the segment (16) of the depot body (14).

8. The active substance depot according to any one of the preceding claims, **characterized in that** the depot body (14) comprises two segments (16) made of the plastic material, between which a metal sleeve (18) is disposed, the free end thereof being bonded to the segments (16) in a gas-tight manner, that the at least one active substance carrier (12) is secured in the metal sleeve (18), and that the metal sleeve (18) acts as a heat transfer element and transfers the body heat of the user to the active substance carrier (20) during use, such that the substance stored in the active substance carrier (20) is released to the air drawn through the flow channel (40) by the user.

9. The active substance depot according to claim 8, **characterized in that** the two segments (16) of the depot body (14) made of the plastic material each comprise an opening (46) connected to the flow channel (40), each sealed by a sealing foil (48) bonded to the depot body (14) in a gas-tight manner by means of heat sealing, said foil being removed or punctured immediately prior to use.

## Revendications

1. Dépôt de matière active pour une installation d'inhalation (10) comprenant un corps de dépôt (14) qui est scellé de manière étanche à l'air vis-à-vis de l'extérieur avant utilisation et qui comporte au moins une section (16) faite d'une matière plastique, un canal d'écoulement (40) à desceller pour pouvoir l'utiliser, et au moins un support de matière active (20) qui est prévu dans le canal d'écoulement (40) et dans lequel est stockée au moins une substance volatile passant au moins en partie en phase gazeuse lorsqu'elle est chauffée, le support de matière active (20) devant être chauffé pour le dégagement de la substance, la matière plastique, dont la section (16) du corps de dépôt (14) est faite, étant un polymère, et la section (16) du corps de dépôt (14), faite dudit polymère, présentant une ouverture (46) reliée au canal d'écoulement (40), qui est scellée par une feuille (48) constituée d'un deuxième matériau et pouvant être retirée ou perforée directement avant utilisation, **caractérisé en ce que** le polymère appartient au groupe des copolymères cyclo-oléfines.

2. Dépôt de matière active selon la revendication 1, **caractérisé en ce que** le polymère présente une perméabilité à la vapeur d'eau selon DIN 53122-2 (à une température ambiante de 23°C et une humidité relative de 85 %) qui se situe dans une plage de 0,02 à 0,05 g mm/mm² d, de préférence dans une plage de 0,03 à 0,04 g mm/m² d, de manière particulièrement préférée à 0,035 g mm/mm².

3. Dépôt de matière active selon la revendication 1, **caractérisé en ce que** la matière plastique, dont la section (16) du corps de dépôt (14) est faite, est résistante à l'hydrolyse, aux acides, aux lessives et aux solvants organiques polaires.

4. Dépôt de matière active selon la revendication 1, 2 ou 3, **caractérisé en ce que** la résistance aux chocs du polymère, dont la section (16) du corps de dépôt est faite, se situe selon ISO 179/1eU (éprouvette 80 mm x 10 mm x 4 mm, sens de la frappe - petit côté, éprouvette non entaillée) dans une plage de 10 à 25 kJ/m², de préférence dans une plage de 15 à 20 kJ/m², de manière particulièrement préférée à 15 kJ/m².

5. Dépôt de matière active selon la revendication 1, 2 ou 3, **caractérisé en ce que** la résistance au choc sur barreau entaillé du polymère, dont la section (16) du corps de dépôt (14) est faite, se situe selon ISO 179/1eA (éprouvette 80 mm x 10 mm x 4 mm, sens de la frappe - petit côté, éprouvette présentant une entaille d'un rayon de 0,25 mm) dans une plage de 3,0 à 1,0 kJ/m², de préférence dans une plage de 1,6 à 2,6 kJ/m², de manière particulièrement préférée à 1,8 kJ/m².

6. Dépôt de matière active selon l'une quelconque des revendications précédentes, **caractérisé en ce que** l'ouverture (46) ménagée sur la section (16) du corps de dépôt (14) faite du polymère est scellée à l'aide d'une feuille métallique (48) reliée au polymère de manière étanche au gaz par thermo-scellage, laquelle peut être retirée ou perforée directement avant utilisation.

7. Dépôt de matière active selon la revendication 6, **caractérisé en ce que** la feuille métallique, de préférence une feuille d'aluminium (48), présente une épaisseur de matériau dans une plage de 20 à 40 µm, de préférence une épaisseur de matériau dans une plage de 25 à 35 µm, de manière particulièrement préférée une épaisseur de matériau de 30 µm et est pourvue sur une de ses faces plates d'une couche de laque de thermo-scellage, au moyen de laquelle la feuille métallique (48) est reliée de manière étanche au gaz au polymère de la section (16) du corps de dépôt (14).

8. Dépôt de matière active selon l'une quelconque des revendications précédentes, **caractérisé en ce que** le corps de dépôt (14) comprend deux sections (16) faites de la matière plastique, entre lesquelles est agencé un manchon métallique (18) dont les extrémités libres sont reliées de manière étanche au gaz aux sections (16), **en ce que** le ou les supports de matière active (12) sont retenus dans le manchon métallique (18), et **en ce que** le manchon métallique (18) sert d'élément de transfert de chaleur et, lors de l'utilisation, transfère la chaleur corporelle de l'utilisateur sur le support de matière active (20), de telle manière que la substance stockée dans le support de matière active (20) soit dégagée dans l'air aspiré par l'utilisateur à travers le canal d'écoulement (40).

9. Dépôt de matière active selon la revendication 8, **caractérisé en ce que** les deux sections (16) du corps de dépôt (14) faites de matière plastique présentent chacune une ouverture (46) reliée au canal d'écoulement (40), lesquelles sont scellées chacune par une feuille de scellement (48) reliée de manière étanche au gaz par thermo-scellage à la section (16) respective du corps de dépôt (14) et pouvant être retirée ou perforée directement avant utilisation.
